# DEMANDE DE BREVET EUROPEEN

(11) **EP 0 850 609 A1**
(43) Date de publication de la demande: **01.07.1998**
(21) Numéro de dépôt: 97403140.3
(22) Date de dépôt: 23.12.1997
(51) Int. Cl.: A61F 2/40

(54) **Prothèse humérale à tête mobile**

(30) Priorité: 30.12.1996 FR 9616203
(71) Demandeur: Aesculap, 52000 Chaumont (FR)
(72) Inventeur: Apoil, André, 78200 Mantes La Jolie (FR)
(74) Mandataire: Robert, Jean-Pierre

(57) **Abrégé**

L'invention concerne une prothèse humérale comportant une tige d'ancrage (1) dont une extrémité (4) est pourvue d'un plateau (5) en saillie duquel est disposée une rotule (11) sur laquelle est montée une tête (13) librement pivotante. Le plateau (5) forme une butée angulaire limitant le pivotement de la tête (13) sur la rotule (11).

## Description

La présente invention concerne une prothèse humérale à tête mobile.

Une prothèse humérale comporte principalement une tige d'ancrage ayant un corps allongé pénétrant dans le canal huméral pour s'y ancrer, la tête humérale ayant été préalablement réséquée. Cette tige d'ancrage possède une extrémité en saillie de l'humérus, à laquelle est raccordée une tête de forme sensiblement hémisphérique se substituant à la tête humérale naturelle.

Selon le type de pathologie rencontré, différents types de prothèse sont préconisés, parmi lesquels on distingue principalement deux grands familles.

Dans un premier type de prothèse, la tête de la prothèse est solidaire de la tige d'ancrage et est mobile avec celle-ci dans la cavité articulaire humérale. Sa surface sphérique est alors en appui contre une prothèse glénoïdienne se substituant à la glène naturelle du patient.

Dans un second type de prothèse, la tête de la prothèse est montée sur une pièce sphérique formant rotule solidaire de l'extrémité de la tige d'ancrage. La tête remplit la cavité articulaire humérale à l'intérieur de laquelle elle se loge et vient en appui contre la glène et l'acromion. La rotule de la prothèse confère donc à la tige d'ancrage (et donc à l'humérus) une première amplitude de pivotement selon toutes les directions. Cette amplitude peut-être complétée par la mobilisation de la tête par rapport à la cavité articulaire humérale. L'invention concerne ce second type de prothèse "à tête mobile".

Un inconvénient important de ce genre de prothèse réside dans l'impossibilité quasi systématique dans la pratique de conserver in situ à la fois la mobilité articulaire intraprothétique et l'absence de douleurs. En effet, après la mise en place de la prothèse, il se forme progressivement, à l'extrémité sectionnée de l'humérus, des ramifications osseuses qui, en particulier du fait d'un contact entre la tête et l'os, créent des douleurs et engendrent une limitation de la mobilité.

En vue de remédier à cet inconvénient, on prévoit selon l'invention une prothèse humérale comportant une tige d'ancrage dont une extrémité est pourvue d'un plateau en saillie duquel est disposée une rotule sur laquelle est montée une tête librement pivotante, caractérisée en ce que le plateau forme une butée angulaire limitant le pivotement de la tête sur la rotule.

Le plateau ménagé à l'extrémité de la tige d'ancrage, est situé, in situ, en recouvrement de l'extrémité sectionnée de l'humérus. Il s'oppose ainsi à la formation de ramifications osseuses et de ce fait, évite l'apparition de douleurs au mouvement et préserve la liberté de mouvement de la tige d'ancrage par rapport à la tête. En combinaison avec cette fonction de limitation des ramifications osseuses, la fonction de butée exercée par le plateau, intrinsèquement à la prothèse, permet de limiter les débattements angulaires extrêmes de la tige. On obtient ainsi une meilleure tolérance des mouvements angulaires extrêmes qui sont pris en compte par les moyens de butée de la prothèse.

Ce plateau permet donc d'assurer une mobilité intraprothétique d'amplitude importante alliée à l'absence de douleurs, tout en ne modifiant pas les dispositions anatomiques de l'articulation de l'épaule.

Avantageusement, la tête comportant une cupule extérieure creuse à l'intérieur de laquelle est fixé un insert présentant un logement pour la rotule, l'insert possède une partie en saillie de la cupule formant une butée pour le plateau de la tige d'ancrage lors du pivotement relatif de la tige d'ancrage par rapport à la tête. L'insert, qui est généralement en polyéthylène, assume entièrement le contact de butée entre la tête et le plateau qui, lui est en métal. La cupule extérieure de la tête étant elle-même en métal, on évite ainsi un contact métal-métal de la cupule avec le plateau. Un tel contact serait en effet trop rigide et conduirait en particulier à l'apparition de bruits et à la dispersion de particules métalliques se détachant de la cupule et du plateau.

Avantageusement encore, l'articulation de la tête sur la rotule présente un jeu. Ce jeu, par la liberté de mouvement supplémentaire qu'il procure, permet d'éviter la création de fibroses entre la tête et le plateau.

Dans un mode de réalisation, le logement ménagé dans l'insert pour la rotule possède, selon une direction sensiblement parallèle à l'axe de révolution de la tête, une portion cylindrique créant le jeu précité.

D'autres caractéristiques et avantages de l'invention apparaîtront à la lecture de la description qui suit d'un de ses modes de réalisation donné à titre d'exemple non limitatif.

Il sera fait référence aux dessins annexés, parmi lesquels :
- la figure 1 est une vue de profil partiellement en coupe d'une prothèse humérale conforme à l'invention ;
- la figure 2 est une vue de face de l'extrémité de la tige d'ancrage seule ;
- la figure 3 est une vue de détail de la zone III de la figure 1 ;
- la figure 4 est une vue analogue à la figure 1 illustrant une configuration d'adduction maximum (dans l'articulation intraprothétique) ;
- la figure 5 est une vue analogue à la figure 1 illustrant une configuration d'abduction maximum (dans l'articulation intraprothétique).

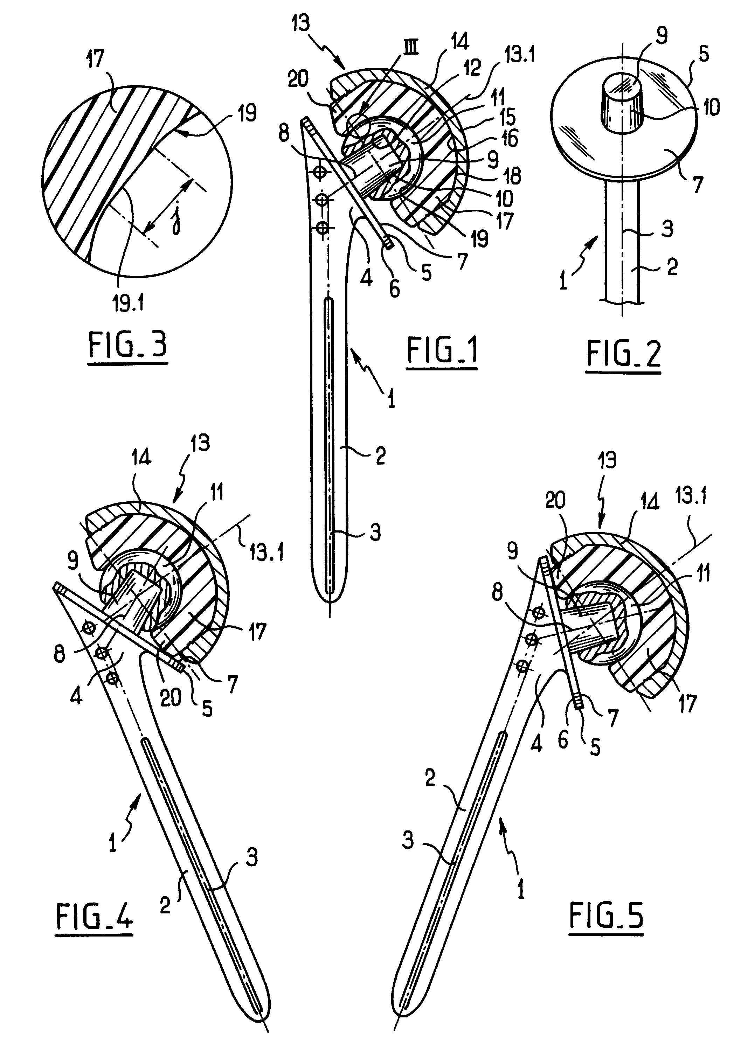

En référence aux figures, et en particulier à la figure 1, la prothèse humérale à tête mobile selon l'invention comporte une tige d'ancrage 1 comportant un corps allongé 2 d'axe 3 destiné à être inséré dans le canal osseux de l'humérus (non représenté) pour s'y ancrer. La constitution de ce corps de tige est bien connue par ailleurs et ne sera donc pas davantage décrite.

La tige d'ancrage 1 possède une extrémité supérieure 4 qui est équipée d'un plateau 5. Ce plateau s'étend selon un plan qui forme ici un angle d'environ 35° avec la direction de l'axe 3 du corps de tige 2. Il présente une face inférieure 6 en regard du corps de tige 2 et une face supérieure 7 opposée. Le plateau 5 est sensiblement plus large que le corps de tige 2. Dans l'exemple illustré, il présente la forme d'un disque ayant un axe central de révolution 8.

Lors de l'implantation de la tige d'ancrage 1 dans le canal huméral, le plateau 5 vient en recouvrement du bord découpé de l'humérus pour former une collerette de bridage s'opposant à toute excroissance de l'humérus. On évite ainsi la formation de ramifications osseuses qui pourraient engendrer des douleurs ou gêner la bonne mobilité de la prothèse.

Un pion de fixation 9 solidaire de la tige d'ancrage 1 est disposé en saillie de la face supérieure 7 du plateau 5, selon l'axe 8. Ce pion de fixation présente une face latérale 10 de forme légèrement conique.

L'ensemble de la tige d'ancrage, avec son corps 2, son plateau 5 et son pion de fixation 9, est réalisé en un matériau métallique tel que de l'acier inoxydable, un alliage de titane, ou un alliage cobalt-chrome.

Une pièce 11 de forme sensiblement sphérique, formant une rotule, est rigidement fixée sur le pion de fixation 9. La rotule 11 présente à cet effet un logement 12 ayant une face latérale conique de forme et de dimension correspondant à celles de la face 10 du pion de fixation 9. La rotule 11 est ainsi emmanchée à force sur le pion 9, de façon à créer un coincement des faces coniques 10 et 12 l'une contre l'autre.

La rotule 11 pourra être réalisée en un matériau métallique tel que de l'acier inoxydable ou un alliage cobalt-chrome ou une céramique.

Une tête articulaire prothétique 13 est montée pour pivoter librement sur la rotule 11. La tête 13 se compose en deux parties principales. Elle comporte ainsi une enveloppe extérieure ou cupule creuse 14 ayant une face extérieure 15 de forme sensiblement sphérique et une face intérieure 16 (de forme sensiblement sphérique également) délimitant un volume intérieur. Cette cupule 14 peut être réalisée en un matériau métallique tel que de l'acier inoxydable ou un alliage chrome-cobalt. In situ, elle remplit la cavité articulaire et vient en appui contre la glène et l'acromion.

La tête 13 comporte de plus un noyau ou insert 17 partiellement logé dans le volume intérieur de la cupule 14 et rigidement fixé à celle-ci. Cet insert présente une face extérieure 18 sensiblement sphérique, en contact avec la face intérieure 16 de la cupule 14, et, à l'opposé de cette face 18, un logement 19 de forme sensiblement sphérique, recevant la rotule 11. Ce logement 19 épouse la rotule 11 sur une portion légèrement supérieure à une hémisphère, de sorte qu'il assure une rétention de la rotule 11. L'insert 17 est réalisé en une matière possédant un faible coefficient de frottement et "biofonctionnel" avec le matériau constitutif de la rotule 11. Il est en particulier possible d'utiliser une matière plastique telle que du polyéthylène à ultra haut poids moléculaire. L'insert 17, ainsi interposé entre la rotule 11 de la tige d'ancrage 1 et la cupule 14, assure une liaison silencieuse et à faible frottement de la tête 13 avec la rotule 11. Il exerce en outre une fonction d'absorption des chocs entre ces deux éléments.

Comme cela est visible à la figure 3, le logement 19 de l'insert 17 possède, selon une direction sensiblement parallèle à l'axe de révolution 13.1 de la tête 13, une portion cylindrique 19.1 de débattement axial. Cette portion de débattement crée, entre la rotule 11 et l'insert 17, un jeu selon une direction parallèle à l'axe 13.1. Ce jeu procure une liberté de mouvement supplémentaire en translation entre la tête 13 et la tige d'ancrage 1. Cette liberté de mouvement permet d'éviter la formation de fibroses entre le plateau 5 et la tête 13. On a référencé en "j" la hauteur de la portion cylindrique 19.1 correspondant au jeu. On pourra avantageusement choisir un jeu de l'ordre de 2mm. Il est à noter que ce jeu est limité du fait du caractère rétentif du logement 19.

Par ailleurs, l'insert 17 déborde légèrement de l'enveloppe 14 et possède ainsi une partie 20 qui s'étend en saillie de l'enveloppe 14. Cette partie en saillie forme une butée pour le plateau 5 lors des pivotements extrêmes de la tige d'ancrage par rapport à la tête 13.

Pour illustrer cette fonction de butée exercée par le plateau 5 et la partie en saillie 20 de l'insert 17, on a illustré respectivement aux figures 4 et 5 les configurations d'adduction et d'abduction intraprothétiques maximums de la prothèse. On constate que la face supérieure 7 du plateau 5 vient, dans ces configurations extrêmes, en butée contre la partie en saillie 20 de l'insert 17. Bien entendu cette fonction de butée s'exerce de la même manière dans toute configuration extrême de pivotement autour d'un axe quelconque.

Comme mentionné précédemment, le plateau 5 exerce, outre cette fonction de butée une première fonction essentielle de prévention de la formation d'éventuelles ramifications osseuses à l'extrémité de l'humérus. On comprend que de telles ramifications pourraient en particulier restreindre anormalement la mobilité de la liaison rotule entre la tige d'ancrage et la tête.

Le plateau 5 permet donc de définir de façon certaine et viable les débattements angulaires maximums de la tige d'ancrage par rapport à la tête.

L'invention n'est pas limitée au mode de réalisation qui vient d'être décrit, mais englobe au contraire toute variante reprenant, avec des moyens équivalents ses caractéristiques essentielles.

## Revendications

1. Prothèse humérale comportant une tige d'ancrage (1) dont une extrémité (4) est pourvue d'un plateau (5) en saillie duquel est disposée une rotule (11) sur laquelle est montée une tête (13) librement pivotante, caractérisée en ce que le plateau (5) forme une butée angulaire limitant le pivotement de la tête (13) sur la rotule (11).

2. Prothèse selon la revendication 1, caractérisée en ce que, la tête (13) comportant une cupule extérieure creuse (14) à l'intérieur de laquelle est fixé un insert (17) présentant un logement (18) pour la rotule (11), l'insert (17) possède une partie (20) en saillie de la cupule (13) qui vient en butée contre le plateau (5) de la tige d'ancrage (1) lors du pivotement relatif de la tige d'ancrage (1) par rapport à la tête (13).

3. Prothèse selon l'une des revendications précédentes, caractérisée en ce que l'articulation de la tête (13) sur la rotule (11) présente un jeu.

4. Prothèse selon les revendications 2 et 3, caractérisée en ce que le logement (18) ménagé dans l'insert (17) pour la rotule (11) possède, selon une direction sensiblement parallèle à l'axe de révolution (13.1) de la tête (13), une portion cylindrique (19) créant le jeu précité.
